Europäisches Patentamt

European Patent Office

Office européen des brevets

(19)

(11) Veröffentlichungsnummer: **0 472 831 A1**

# (12) EUROPÄISCHE PATENTANMELDUNG

(21) Anmeldenummer: **91109588.3**

(51) Int. Cl.⁵: **G01N 1/10, G21F 7/005**

(22) Anmeldetag: **11.06.91**

| | |
|---|---|
| (30) Priorität: **27.07.90 DE 4023839** | **W-3000 Hannover 1(DE)** |
| (43) Veröffentlichungstag der Anmeldung: **04.03.92 Patentblatt 92/10** | (72) Erfinder: **Zeh, Horst** **Kolberger Strasse 19 b** **W- 7500 Karlsruhe 1(DE)** |
| (84) Benannte Vertragsstaaten: **CH DE FR GB LI SE** | |
| (71) Anmelder: **Deutsche Gesellschaft für Wiederaufarbeitung von Kernbrennstoffen mbH Hamburger Allee 4 Postfach 1407** | (74) Vertreter: **König, Norbert, Dipl.-Phys. Dr. et al Patentanwälte Leine & König Burckhardtstrasse 1 W-3000 Hannover 1(DE)** |

(54) **Vorrichtung zum Entnehmen von Gas- und/oder Flüssigkeitsproben aus dem Sicherheitsbehälter von Kernkraftwerken.**

(57) Eine Vorrichtung zum Entnehmen von Gas- und/oder Flüssigkeitsproben aus dem Sicherheitsbehälter von Kernkraftwerken verwendet evakuierte Probenbehältnisse zur Aufnahme der Proben, wobei die Probenbehältnisse pneumatisch in die Entnahmevorrichtung hinein und aus der Vorrichtung heraustransportiert werden. Die Entnahmevorrichtung weist einen Probennehmerkopf (2) auf, der innerhalb des Sicherheitsbehälters installiert ist und der eine Aufnehmerkugel (8) mit einer Ausnehmung (10) zur Aufnahme des Probenbehältnisses (14) aufweist. Die Aufnehmerkugel ist aus einer Aufnahmeposition in eine Befüllposition und umgekehrt mit Hilfe einer pneumatischen Stelleinrichtung drehbar. Die Entnahmevorrichtung weist ferner eine einen Pneumatikzylinder (4) umfassende Befüllvorrichtung (5) auf, die an den Probennehmerkopf (2) angeflanscht ist und die mit dem Probennehmerkopf und damit mit dem Probenbehältnis bei Drehung der Aufnehmerkugel (8) in die Befüllposition in Wirkverbindung bringbar ist zum Befüllen des Probenbehältnisses. Der Pneumatikzylinder (4) weist einen Kolben mit einer Kolbenstange (58) auf, deren Kolbenkopf (64) eine Hohlnadel (66) aufweist, die in der Befüllposition der Aufnehmerkugel durch den selbsttätig schließenden Verschluß (49) des Probenbehältnisses (14) hindurchbewegt wird.

Die Erfindung betrifft eine Vorrichtung zum Entnehmen von Gas- und/oder Flüssigkeitsproben aus dem Sicherheitsbehälter von Kernkraftwerken gemäß Oberbegriff des Anspruchs 1.

Aus der EP-A 0 093 609 ist ein Probennehmersystem für eine Anlage zur Wiederaufarbeitung von abgebrannten Kernbrennstoffen bekannt. Die Probennahme erfolgt mit Hilfe von Probenflaschen, die von einem Träger aufgenommen werden, der pneumatisch zwischen einer Analysestation und einer Probennahmestation transportiert wird. An jeder Probennahmestation ist ein Manipulator zur Aufnahme des Trägers angebracht zum Entfernen der Flasche vom Träger, zum Transport der Flasche zur Probenzapfstelle, an der eine Probe in die Flasche eingefüllt wird, und zum Beladen des Trägers mit der gefüllten Flasche, die dann pneumatisch zur Analysestation rücktransportiert wird.

Durch die DE-PS 37 10 713 ist eine Vorrichtung zum Entnehmen von Proben radioaktiver und/oder toxischer Substanzen aus Verfahrenskomponenten bekannt, die in abgeschirmten, unzugänglichen Zellen angeordnet sind. Die Probennahme erfolgt mit Hilfe von Probenbehältern, die über ein Rohrleitungssystem von einer entfernt von den Verfahrenskomponenten angeordneten Untersuchungsstation zu einer Probennahmestation mit Hilfe eines verfahrbaren Trägers transportiert werden. Der Träger ist mittels eines elektrischen Antriebes gesteuert zwischen der Probennahmestation und der Untersuchungsstation verfahrbar. Leitungen von mehreren Probennahmestellen an den einzelnen Verfahrenskomponenten sind zu einem gemeinsamen Kopf der Probennahmestation geführt, wobei der Träger einen Gegenkopf aufweist und der gemeinsame Kopf und dieser Gegenkopf so ausgebildet sind, daß allein durch geradlinige Verschiebung des Trägers gegenüber dem Kopf die Probenbehälter mit den zugeordneten Leitungen so verbunden werden, daß Proben in die Probenbehälter gefüllt werden.

Die Aufgabe der vorliegenden Erfindung besteht darin, eine Vorrichtung zum Entnehmen von Gas- und/oder Flüssigkeitsproben aus dem Sicherheitsbehälter von Kernkraftwerken anzugeben, mit der auch in einem Störfalle die Entnahme von Proben aus dem Flüssigkeitssumpf und dem Gasraum sicher möglich ist.

Diese Aufgabe wird durch die Erfindung gemäß Anspruch 1 gelöst.

Vorteilhafte und zweckmäßige Weiterbildungen der erfindungsgemäßen Aufgabenlösung sind in den Unteransprüchen gekennzeichnet.

Die erfindungsgemäße Vorrichtung ist unmittelbar vor Ort entweder im Flüssigkeitssumpf eingetaucht angeordnet, um die Probe aus der sie umgebenden Flüssigkeit zu nehmen, oder im anderen Einsatzfall, die Probe aus der sie umgebenden Gasraumatmosphäre zu entnehmen. Es wird dabei ein evakuiertes Probenbehältnis verwendet, dessen Transport von einer außerhalb des Sicherheitsbehälters befindlichen Prüfstation zur Probennahmevorrichtung durch eine Druckluftförderanlage erfolgt, deren Druckluftförderkanal durch die Wandung des Sicherheitsbehälters hindurchgeführt ist.

Die vorgeschlagene Vorrichtung zum Entnehmen von Proben ist ohne Änderung sowohl für die Probennahme aus dem Gasraum als auch aus dem Flüssigkeitssumpf eines Sicherheitsbehälters einsetzbar.

Die erfindungsgemäße Vorrichtung weist einen Probenbehältnisaufnehmer, vorzugsweise in Form einer Kugel auf, die eine Ausnehmung zur Aufnahme des Probenbehältnisses aufweist. Die Aufnehmerkugel ist von einer Aufnahme-/Abgabeposition in eine Befüllposition und umgekehrt drehbar. Der Probenbehältnisverschluß wird gegen einen Anschlagbund mit Dichtlippen beim Drehen in die Befüllposition und beim Befüllen gedrückt, wodurch ein Verschleppen von kontaminiertem Probemedium über die Ausnehmung in den Druckluftförderkanal verhindert wird. Die Drehung der Aufnehmerkugel in die Befüllposition und zurück erfolgt durch einen pneumatisch betätigten Stellantrieb. Zur Befüllung des Probenbehältnisses wird dessen Verschluß von einer Hohlnadel durchstoßen, und die Probemenge wird aus der die Entnahmevorrichtung umgebenden Flüssigkeits- oder Gasatmosphäre über die zur Hohlnadel führenden Öffnungen und Kanäle in das evakuierte Probenbehältnis eingesaugt.

Die für das Einführen und Abziehen der Hohlnadel erforderliche Hubbewegung wird von einem am Probennehmerkopf angeflanschten Pneumatikzylinder erzeugt. Die Hohlnadel ist am Kopfstück einer Kolbenstange des Pneumatikzylinders befestigt. Nach Beendigung des Befüllvorganges wird der Probenbehältnisaufnehmer bzw. wird die Aufnehmerkugel mit dem in ihr befindlichen gefüllten Probenbehältnis wieder in die Aufnahme- bzw. Abgabeposition zurückgedreht, und das Probenbehältnis wird dann pneumatisch aus dem Sicherheitsbehälter herausgefördert und zur Prüfstation weitertransportiert.

Die erfindungsgemäße Probenentnahmevorrichtung ist materialmäßig und konstruktiv so ausgelegt, daß es den besonderen Umgebungsbedingungen und Sicherheitsvorgaben während und nach einem Störfall im Reaktor Rechnung trägt. Die Probennahmevorrichtung wird dabei im wesentlichen hinsichtlich folgender Kriterien ausgelegt:

Explosionen und Druckwellen im Rahmen der vom Sicherheitsbehälter zu beherrschenden Stärke; hohes radioaktives Strahlungsfeld; Behälterinnendruck bis 6 bar; Behälteratmosphäre bestehend aus Wasserdampf, Wasserstoff und Edelgasen; Temperaturen bis 140°C; schwebende Feststoffpartikel im Flüssigkeitssumpf. Außerdem muß die Vorrichtung fernbedienbar ausgeführt sein.

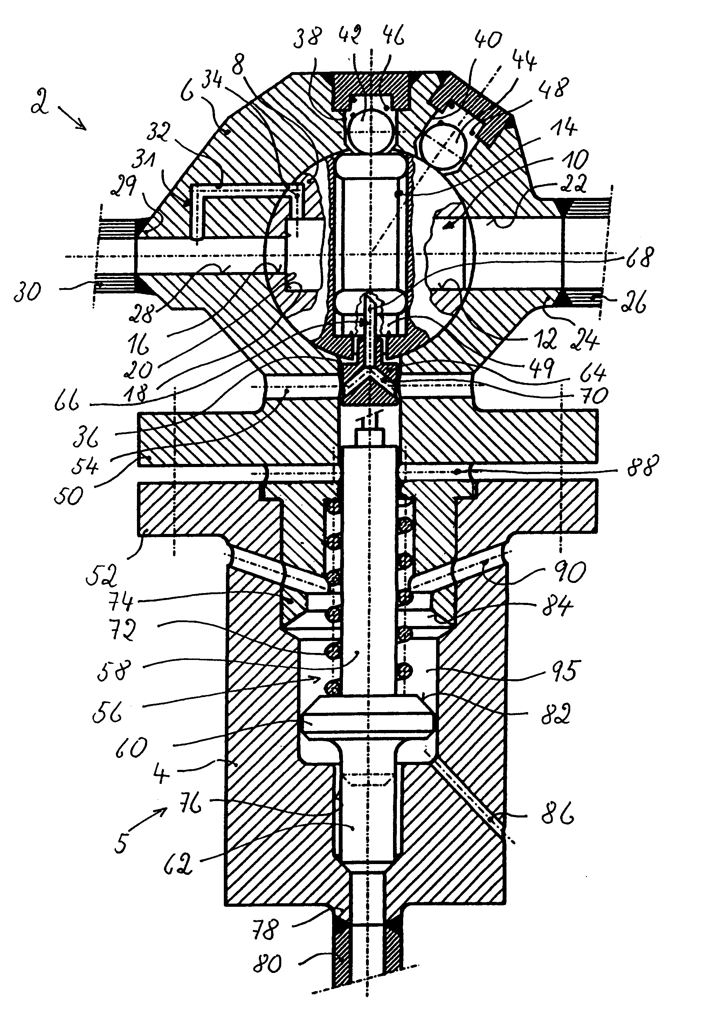

Die Erfindung soll nachfolgend anhand der beigefügten Zeichnung, die ein Ausführungsbeispiel der erfindungsgemäßen Vorrichtung zeigt, näher erläutert werden.

Die Zeichnung zeigt eine in einem Sicherheitsbehälter (nicht dargestellt) von Kernkraftwerken angeordnete Vorrichtung zum Entnehmen von Gas- und/oder Flüssigkeitsproben mit einem Probennehmerkopf 2 und einer am Probennehmerkopf angeflanschten, einen Pneumatikzylinder 4 aufweisenden Befüllvorrichtung 5.

Der Probennehmerkopf 2 weist ein Kopfgehäuse 6 auf, in dem eine Aufnehmerkugel 8 drehbar gelagert ist, die von einem Stellantrieb (nicht dargestellt) drehbar und einstellbar ist.

Die Aufnehmerkugel 8 weist eine diametrale Kugelausnehmung 10 auf, die aus zwei unterschiedlich großen Bohrungen, einer größeren, längeren Aufnahmebohrung 12 zur Aufnahme einer evakuierten Probeflasche 14 und einer kleineren, zentral zur größeren Bohrung 12 angeordneten, kürzeren Zirkulationsbohrung 16 besteht, wobei ein ringförmiger Anschlagbund 18 gebildet ist, der an der Übergangskante zur Zirkulationsbohrung 16 als Lippendichtung 20 ausgebildet ist.

Die Aufnahmebohrung 12 ist mit einer im Kopfgehäuse 6 ausgebildeten gleich großen Transportbohrung 22 ausrichtbar, die einen Anschluß 24 zum Anschluß an einen pneumatischen Transportkanal 26 aufweist, über den die Probenflasche 14 von einer außerhalb des Sicherheitsbehälters befindlichen Prüfstation in die Aufnahmebohrung 12 zur Probennahme und wieder zurück zur Prüfstation transportiert wird.

Die Zirkulationsbohrung 16 ist mit einem im Kopfgehäuse ausgebildeten Zirkulationskanal 28 ausrichtbar, der über einen Zirkulationsanschluß 29 an eine Zirkulationsleitung 30 der pneumatischen Transporteinrichtung angeschlossen ist. Der Zirkulationskanal 28 kann über einen Bypasskanal 31 mit der Aufnahmebohrung 12 in Verbindung gebracht werden. Der Bypasskanal 31 besteht aus einem Kopfgehäusekanal 32 und einem in der Aufnahmekugel 8 ausgebildeten, mit dem Kopfgehäusekanal ausrichtbaren Kugelkanal 34, der in die Aufnahmebohrung 12 mündet. Der Bypasskanal 31 dient zur Entlüftung eines sich beim Beschicken der Kugelausnehmung 10 mit der Probenflasche vor dem Anschlagbund 18 aufbauenden Luftpolsters.

Über den Transportkanal 26 erfolgt der Hin- und Rücktransport der Probenflasche 14. Die Förderluft zirkuliert über die entgegengesetzt dazu am Zirkulationsanschluß 29 angeschlossene Zirkulationsleitung 30.

Das Kopfgehäuse 6 weist ferner einen Befüllkanal 36 und diametral gegenüber dem Befüllkanal eine erste Lagerbohrung 38 und dazu versetzt in Richtung der Transportbohrung eine weitere Lagerbohrung 40 auf. In den Lagerbohrungen sind Gleitkugeln 42, 44 angeordnet, die mit Hilfe von Federn 46, 48 radial einwärts belastet sind. Diese federbelasteten Gleitkugeln 42, 44 stellen sicher, daß die Probenflasche 14 stets mit ihrem Verschluß 49 gegen die Lippendichtung 20 gepreßt wird und somit sowohl beim Drehen der Aufnehmerkugel 8 mit der darin befindlichen Probenflasche in die Befüllposition als auch während des Befüllvorganges kein Probenmedium in die Kugelausnehmung 10 und damit in den Transportkanal 26 gelangen kann.

Der Befüllkanal 36 durchsetzt einen Kopfgehäuseflansch 50, der mit einem Zylinderflansch 52 des Pneumatikzylinders 4 verbunden ist. In dem Befüllkanal 36 führen mehrere, vorzugsweise vier jeweils um 90° versetzte, im Halsbereich des Probennehmerkopfes 2 ausgebildete Zulaufbohrungen 54 für das Probenmedium.

Im Pneumatikzylinder 4 ist ein Kolben 56 mit Kolbenstange 58, Kolbenbund 60 und Kolbenzapfen 62 geführt. Die Kolbenstange weist einen Kolbenstangenkopf 64 auf, der in den Befüllkanal 36 hineinbewegbar ist bei Druckbeaufschlagung des Kolbens. Der Kolbenstangenkopf 64 weist eine Hohlnadel 66 auf, deren Nadelkanal 68 mit im Kolbenstangenkopf 64 ausgebildeten, den Zulaufbohrungen 54 zugeordnete und mit diesen in Verbindung bringbare Zulaufkanäle 70 in Verbindung steht. Die Hohlnadel 66 liegt in Außerbetriebsstellung geschützt im Befüllkanal 36 im Bereich des Kopfgehäuseflansches 50. Die Hohlnadel wird über die Zulaufbohrungen 54 und die Zulaufkanäle 70 mit Probenmedium versorgt.

Die Kolbenstange 58 ist schwimmend gelagert und bedarf keiner Abdichtung. Sie wird ständig vom Probenmedium umspült.

Eine Rückstellfeder 72, die sich an einer die Kolbenstange führenden Zylindergehäusebuchse 74 abstützt, welche Teil des Kopfgehäuses 6 ist, wirkt auf den Kolbenbund 60, der sich zwischen der Kolbenstange 58 und dem Kolbenzapfen 62 befindet. Der Kolbenzapfen 62 befindet sich in einer Zylinderbohrung 76, die über einen Druckluftanschluß 78 und eine Druckluftleitung 80 an eine Druckluftversorgung angeschlossen ist. Die Zirkulations- und Druckluftleitungen sind durch die Wandung des Sicherheitsbehälters zu den entsprechenden Steuer- und Regelstationen geführt.

Der Kolbenbund 60 weist kolbenstangenseitig eine angeschrägte ringförmige Dichtfläche 82 auf, die sich gegen eine komplementär angeschrägte Dichtfläche 84 der Zylindergehäusebuchse 74 abdichtend anlegt, wenn die Hohlnadel 66 in der Befüllposition in die Probeflasche 14 eingeführt wird; hierdurch dichtet der Kolbenbund den Bereich der Zulaufbohrungen 54 und der Zulaufkanäle 70 gegenüber der eingesteuerten Druckluft ab.

Das Einführen der Hohlnadel 66 in den Verschluß 49 der Probenflasche 14 zum Befüllen derselben erfolgt durch Druckbeaufschlagung des in der Zylinderbohrung 76 sitzenden Kolbenzapfens 62 über den Druckluftanschluß 78 und die dadurch bewirkte Hubbewegung der Kolbenstange 58 in Richtung Probennehmerkopf 2 gegen die Wirkung der Rückstellfeder 72. Dabei durchdringt die Hohlnadel 66 den selbstdichtend ausgebildeten Verschluß 49 der Probenflasche.

Die Druckentlüftung nach Beendigung des Befüllvorganges zur Rückstellung der Kolbenstange 58 durch die Rückstellfeder und damit zum Abziehen der Hohlnadel aus dem Verschluß der Probenflasche sowie das Freispülen der Druckkammer von Ablagerungen erfolgt über eine Entlüftungsbohrung 86. Beim Abziehen der Hohlnadel dichtet der Flaschenverschluß selbsttätig ab; er besteht aus einem Gummimaterial.

Im Gehäuse 6 des Probennehmerkopfes 2 sind im Bereich zwischen Kopfgehäuseflansch 50 und Zylinderflansch 52 vier Spülbohrungen 88 und im Pneumatikzylinder 4 vier Entlüftungsbohrungen 90 vorgesehen. Über diese Spül- und Entlüftungsbohrungen 88 und 90 wird die Vorrichtung mit Probenmedium geflutet, gespült und nach der Druckluftbeaufschlagung entlüftet.

Nachfolgend soll die Funktionsweise der oben beschriebenen Vorrichtung erläutert werden.

Über eine außerhalb des Sicherheitsbehälters (nicht dargestellt) befindliche Beschickungsstation (nicht dargestellt) wird eine evakuierte Probenflasche 14 in den Drucklufttransportkanal 26 eingeschleust und zur Probennahmevorrichtung, die im Flüssigkeitssumpf und/oder in der Gasatmosphäre des Sicherheitsbehälters installiert ist, befördert. Im Probennehmerkopf 2 wird die Probenflasche in der Kugelausnehmung 10 der Aufnehmerkugel 8 am Anschlagbund 18 aufgefangen. Die im Transportkanal 26 befindliche Förderluft zirkuliert dabei über die Zirkulationsleitung 30, in die über den Bypasskanal 31 auch die Entlüftung des sich vor der Lippendichtung 20 am Anschlagbund aufbauenden Luftpolsters erfolgt.

Nach dem Auffangen der Probenflasche 14 in der Kugelausnehmung 10 der Aufnehmerkugel 8 wird diese durch einen pneumatischen Stellantrieb (nicht dargestellt) um 90° in die Befüllposition gedreht. Der Verschluß 49 der Probenflasche 14 befindet sich danach in der Öffnung des Befüllkanals 36 des Kopfgehäuses 6 und ist für die Hohlnadel 66 zum Eindringen zugänglich, vgl. Zeichnung.

Durch eine stoßartige Druckluftbeaufschlagung des Pneumatikzylinders 4 wird die die Hohlnadel 66 tragende Kolbenstange 58 mit einem kurzen Hub in Richtung Probenflasche 14 bewegt, und die Hohlnadel 66 dringt durch den Gummiverschluß 49 in die Probenflasche ein. Bei der stoßartigen Hubbewegung werden die mit Probemedium gefüllten Zulaufkanäle 70 im Kolbenstangenkopf 64 und die Zulaufbohrungen 54 im Probennehmer-Kopfgehäuse 6 sowie die Hohlnadel 66 selbst gespült, von eventuellen Sedimentationen, die zu Verstopfungen führen könnten, befreit und um die Befüllbohrung eine Verwirbelung / Vermischung des zu analysierenden Probenmediums erzeugt.

Durch die freigespülten Zuläufe kann nunmehr ungehindert das die Probennehmervorrichtung umgebende Probenmedium selbsttätig über die Hohlnadel 66 von der evakuierten Probenflasche 14 innerhalb kürzester Zeit eingesaugt werden.

Während der Probennahme wird durch den Kolbenbund 60 der Bereich der Zuläufe für das Probenmedium von der über den Druckluftanschluß 78 anstehenden Druckluft abgedichtet.

Nach Beendigung des Befüllvorganges und Nachlassen der Druckluftbeaufschlagung (Entlüftung über die Entlüftungsbohrung 86) auf die Kolbenstange 58 wird durch die Wirkung der Rückstellfeder 72 auf den Kolbenbund 60 automatisch die Hohlnadel 66 von der Probenflasche 14 abgezogen, und der selbstdichtende Gummiverschluß 49 (Septum) schließt die Probenmenge sicher in der Probenflasche ein. Die Aufnehmerkugel 8 mit der jetzt gefüllten Probenflasche 14 wird danach wieder in die ursprüngliche Position (Aufnahmeposition) um 90° zurückgedreht, und die Probenflasche wird mittels Förderluftbeaufschlagung von seiten des Zirkulationsleitungsanschlusses 29 über den Transportkanal 26 zur Prüfstation außerhalb des Sicherheitsbehälters befördert.

## Patentansprüche

1. Vorrichtung zum Entnehmen von Gas- und/oder Flüssigkeitsproben aus Sicherheitsbehältern von Kernkraftwerken mit Hilfe eines evakuierten Probenbehältnisses, das pneumatisch in die Vorrichtung hinein und aus der Vorrichtung heraus transportierbar ist, **gekennzeichnet durch**

   einen innerhalb des Sicherheitsbehälters angeordneten Probennehmerkopf (2) mit einem Kopfgehäuse (6), in dem ein mit einer Ausnehmung (10) zur Aufnahme des Probenbehältnisses (14) versehener Probenbehältnis-Aufnehmer (8) angeordnet ist, der in eine Probenbehältnis-Aufnahme / Abgabeposition und eine Probenbehältnis-Befüllposition bewegbar ist, und

eine mit dem Probennehmerkopf lösbar verbundene Befüllvorrichtung (5), die mit dem Probennehmerkopf (2) und mit dem Probenbehältnis (14) bei Bewegung des Aufnehmers (8) in die Befüllposition in Wirkverbindung bringbar ist zum Befüllen des Probenbehältnisses.

2. Vorrichtung nach Anspruch 1, **dadurch gekennzeichnet**, daß der Probenbehältnis-Aufnehmer (8) als Aufnehmerkugel ausgebildet ist, die in die jeweiligen Positionen mit Hilfe einer Stellvorrichtung drehbar ist.

3. Vorrichtung nach Anspruch 1 oder 2, **dadurch gekennzeichnet**, daß die Ausnehmung (10) diametral ausgebildet ist und aus einer ersten größeren, längeren Aufnahmebohrung (12) zur Aufnahme des Probenbehältnisses (14) und einer zweiten kleineren, kürzeren Zirkulationsbohrung (16) besteht, die zentral zur ersten Bohrung (12) angeordnet ist, derart, daß ein ringförmiger Anschlagbund (18) für das Probenbehältnis (14) gebildet wird.

4. Vorrichtung nach Anspruch 3, **dadurch gekennzeichnet**, daß an der Übergangskante von der Aufnahmebohrung (12) zur Zirkulationsbohrung (14) eine Lippendichtung (20) angeordnet ist.

5. Vorrichtung nach einem der vorhergehenden Ansprüche, **dadurch gekennzeichnet**, daß die Aufnahmebohrung (12) mit einer im Kopfgehäuse (6) ausgebildeten, gleich großen Transportbohrung (22) ausrichtbar ist, die an einen pneumatischen Transportkanal (26) angeschlossen ist, über den das Probenbehältnis (14) von einer außerhalb des Sicherheitsbehälters befindlichen Station in die Aufnahmebohrung (12) zur Probennahme und wieder zurück zur Station transportierbar ist.

6. Vorrichtung nach Anspruch 3 oder 4, **dadurch gekennzeichnet**, daß die Zirkulationsbohrung (16) mit einem im Kopfgehäuse (6) ausgebildeten Zirkulationskanal (28) ausrichtbar ist, der an eine Zirkulationsleitung (30) der Förderluft der pneumatischen Transporteinrichtung angeschlossen ist.

7. Vorrichtung nach einem der vorhergehenden Ansprüche, **dadurch gekennzeichnet**, daß der Zirkulationskanal (28) in der Aufnahmeposition der Aufnehmerkugel (8) über einen Bypasskanal (31) mit der Aufnahmebohrung (12) in Verbindung steht.

8. Vorrichtung nach einem der vorhergehenden Ansprüche, **dadurch gekennzeichnet**, daß das Kopfgehäuse (6) einen Befüllkanal (36) aufweist, mit dem die Ausnehmung (10) der Aufnehmerkugel (8) in der Befüllposition in Verbindung steht.

9. Vorrichtung nach Anspruch 8, **dadurch gekennzeichnet**, daß das Kopfgehäuse (6) diametral gegenüber dem Befüllkanal (36) und versetzt dazu in Richtung der Transportbohrung (22) Lagerbohrungen (38, 40) aufweist, in denen Gleitkugeln (42, 44) angeordnet sind, die von Druckfedern (46, 48) radial einwärts belastet sind.

10. Vorrichtung nach einem der vorhergehenden Ansprüche, **dadurch gekennzeichnet**, daß die Befüllvorrichtung (5) einen Pneumatikzylinder (4) umfaßt, in dem ein mit Druckluft betätigbarer Kolben (56) angeordnet ist, der eine Kolbenstange (58) aufweist, die in den Befüllkanal (36) des Kopfgehäuses (6) hineinbewegbar ist und einen Kolbenstangenkopf (64) mit einer Hohlnadel (66) aufweist, die in das Probenbehältnis (14) einführbar ist und über die das Probenbehältnis mit Probenmedium befüllbar ist.

11. Vorrichtung nach Anspruch 10, **dadurch gekennzeichnet**, daß der Nadelkanal (68) der Hohlnadel (66) mit im Kolbenstangenkopf (64) ausgebildeten Zulaufkanälen (70) in Verbindung steht, die wiederum mit im Probennehmerkopfgehäuse (6) ausgebildeten Zulaufbohrungen (54) für das Probenmedium verbunden sind.

12. Vorrichtung nach Anspruch 10 oder 11, **dadurch gekennzeichnet**, daß der Kolben (56) von einer Rückstellfeder (72) beaufschlagt ist.

13. Vorrichtung nach einem der vorhergehenden Ansprüche, **dadurch gekennzeichnet**, daß das Probenbehältnis (14) eine Probenflasche ist, deren Verschluß (49) von der Hohlnadel (66) zum Befüllen durchstoßen wird.

14. Vorrichtung nach Anspruch 13, **dadurch gekennzeichnet**, daß der Verschluß (49) selbstdichtend ausgebildet ist.

15. Vorrichtung nach einem der vorhergehenden Ansprüche, **dadurch gekennzeichnet**, daß die Kolbenstange (58) schwimmend gelagert und vom Probenmedium umspült ist.

16. Vorrichtung nach einem der vorhergehenden Ansprüche, **dadurch gekennzeichnet**, daß der Kolben (56) in der Befüllposition mit dem Kolbenbund (60) an einer Dichtfläche (84) anliegt zur Trennung der über die Druckluftleitung eingesteuerten anstehenden Druckluft von in den Zulaufbohrungen (54) und Zulaufkanälen (70) befindlichem Probenmedium.

17. Vorrichtung nach einem der vorhergehenden Ansprüche, **dadurch gekennzeichnet**, daß im Probennehmerkopfgehäuse (6) Spülbohrungen (88) und im Pneumatikzylinder (4) Entlüftungsbohrungen (90) ausgebildet sind, über die bei Druckbeaufschlagung des Kolbens (56) und vor dem Eindringen der Hohlnadel (66) in den Verschluß (49) der Probenflasche (14) eine Spülung der Zulaufbohrung (54) des Kopfgehäuses (6) und der Zulaufkanäle (70) des Kolbenstangenkopfes (64) sowie des Nadelkanals (68) der Hohlnadel (66) erfolgt.

18. Vorrichtung nach einem der vorhergehenden Ansprüche, **dadurch gekennzeichnet**, daß eine Entlüftungsbohrung (86) in der Druckkammer (95) des Pneumatikzylinders (4) zur Druckentlüftung und zum Freispülen im hinter dem Kolbenbund liegenden Zylinderbereich angeordnet ist.

19. Vorrichtung nach Anspruch 16, **dadurch gekennzeichnet**, daß die Dichtfläche (84) an einer im Zylinder (4) eingesetzten, einen Teil des Kopfgehäuses (6) bildenden Buchse (74) ausgebildet ist.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X,A | DE-A-3 438 303 (WIEDERAUFARBEITUNGSANLAGE KARLSRUHE BETRIEBSGESELLSCHAFT) <br> * Seite 5, Absatz 4 - Seite 7, Absatz 3 * * Figur * <br> — — — | 1,5,13,14, 2,3,6,8, 10,11 | G 01 N 1/10 <br> G 21 F 7/005 |
| D,A | EP-A-0 093 609 (BRITISH NUCLEAR FUELS) <br> * Seite 3, Zeilen 9 - 24 * * Seite 10, Zeilen 9 - 20 * * Seite 16, Zeilen 5 - 19 * * Figuren 1, 2, 8a * <br> — — — | 1,3,5,13, 14 | |
| A | EP-A-0 078 212 (COMMISSARIAT A L'ENERGIE ATOMIQUE) <br> * das ganze Dokument * <br> — — — | 1,5,8,10, 13,14 | |
| A | GB-A-2 171 515 (WIEDERAUFARBEITUNGSANLAGE KARLSRUHE BETRIEBSGESELLSCHAFT) <br> * das ganze Dokument * <br> — — — | 1,5,13,14 | |
| D,A | DE-C-3 710 713 (DEUTSCHE GESELLSCHAFT FÜR WIEDERAUFARBEITUNG VON KERNBRENNSTOFFEN) <br> * Spalte 2, Zeile 65 - Spalte 6, Zeile 16 * * Figuren 1, 5 * <br> — — — — — | 1,13,14, 17 | |

**RECHERCHIERTE SACHGEBIETE (Int. Cl.5)**

G 01 N
G 21 F

Der vorliegende Recherchenbericht wurde für alle Patentansprüche erstellt

| Recherchenort | Abschlußdatum der Recherche | Prüfer |
|---|---|---|
| Berlin | 19 Dezember 91 | JOHNSON K M |